Europäisches Patentamt

European Patent Office

Office européen des brevets

(11) Publication number: **0 087 898**
**A1**

(12) **EUROPEAN PATENT APPLICATION**

(21) Application number: **83300854.3**

(22) Date of filing: **18.02.83**

(51) Int. Cl.³: **C 07 G 7/00**
**C 12 P 1/00, C 12 N 15/00**
**G 01 N 33/54, C 12 N 5/00**
**//A61K39/00, A61K39/395,**
**C12R1/91**

(30) Priority: **22.02.82 GB 8205134**
**22.02.82 GB 8205135**
**28.04.82 GB 8212329**
**30.04.82 GB 8212634**
**13.07.82 GB 8220320**
**13.07.82 GB 8220332**

(43) Date of publication of application:
**07.09.83 Bulletin 83/36**

(84) Designated Contracting States:
**AT BE CH DE FR GB IT LI LU NL SE**

(71) Applicant: **CARLTON MEDICAL PRODUCTS LIMITED**
**2 Carlton House Terrace**
**London SW1Y 5AR(GB)**

(72) Inventor: **Harris, Henry**
**73 Cumnor Hill**
**Oxford Oxon OX2 9HX(GB)**

(74) Representative: **Evans, David Charles et al,**
**F.J. CLEVELAND & COMPANY 40-43, Chancery Lane**
**London, WC2A 1JQ(GB)**

(54) **Antibodies and antigens useful in the diagnosis and treatment of cancer.**

(57) This invention relates to novel antigens and antibodies, the antibodies being useful in detecting the antigen which is present in the cell membrane of malignant cancer cells. The invention includes a method of testing for malignant cancer cells and pharmaceutical compositions including at least the antibodies of the invention.

"ANTIBODIES AND ANTIGENS USEFUL IN THE DIAGNOSIS AND

TREATMENT OF CANCER"

DESCRIPTION

The present invention relates to monoclonal antibodies which are useful in detecting markers in tumour cells, antigens capable of binding with said antibodies, methods of making said antibodies and antigens, pharmaceutical preparations, and diagnostic tests using said antibodies and antigen.

For some years now the technique of cell fusion has been used to analyse at cellular level, the generic determinants of malignancy which can be defined as the ability of tumour cell to multiply progressively in a genetically compatible immunosuppressed host. When malignant tumour cells are fused with normal cells of the same species, the hybrid cells so formed, so long as they retain something close to the chromosome sets of the two parent cells, usually show suppression of malignancy, but when certain genetic elements derived from the normal parent cell are eliminated from the hybrid this suppression is relieved and the hybrid cells again become malignant.

Matched pairs of such hybrids, one partner being malignant and the other non-malignant, provide excellent screens for detecting markers that are closely associated with malignancy. If one simply compares a malignant cell with normal cells, (the corresponding normal cell is rarely available and may not be known), one may discover many differences between the two, but the great majority of these differences may have nothing to do with the production of the malignant state. If, however, one compares hybrid cells in which malignancy is suppressed with malignant segregants derived from these same hybrids, then it will be clear that markers associated solely with cell multiplication are eliminated since both the suppressed hybrid and the malignant segregant derived from it are assayed as cell cultures multiplying in the same conditions. Any consistent difference between the partners of such matched pairs of hybrid cells has a good chance of being closely linked to whatever it is that confers on a tumour cell the ability to grow progressively in vivo .

These studies have resulted in the identification of three cellular marks of malignancy that have survived extensive testing in screens of this kind. These markers are as follows:-

1. An abnormality in the polysaccharide structure of an apparently dimeric membrane glycoprotein having a sub-unit molecular mass of approximately 100,000. Strong circumstantial evidence was subsequently obtained that this protein has some role in glucose transport.

2. The detection of a monomeric membrane glycoprotein with a molecular mass of about 90,000. This was detected in malignant cells by metabolic labelling with (C14) glucosamine, but could not be detected in normal cells. The amount of this protein made by malignant cells was found to be influenced by the ambient glucose concentration. Since both of these markers were involved in the glucose metabolism, the kinetic parameters of hexose transport were studied in a similar panel of matched pairs of hybrid cells. This study revealed;

- 4 -

3.    That malignancy is systematically linked  to a reduction in the Michaelis constant of the hexose transport system.

As a result of this work the present Applicants have prepared an antigen derived from malignant cells.

According to the present invention there is provided an antigen derived from malignant cells characterised by:

1.    a molecular mass within the range of 340,000 to 400,000,

2.    the ability to bind to the lectin wheat germ agglutinin,

3.    resistance to boiling,

4.    resistance to destruction and to extraction from malignant cells using solvents selected from ethyl alcohol, methanol, xylol and chloroform-methanol mixtures.

The antigen may be further characterised by being destructable on digestion with pronase.  In a particular embodiment of the present invention the antigen may have molecular mass components of 350,000 and 390,000 approximately when determined by sodium deoxycholated extraction of malignant cells and fractionation of the extract by high performance liquid chromatography.

The antigen may be further characterised as glycoproteins having a high hydrocarbon content.

Investigations have shown that malignant cells growing in vitro shed the antigen into the medium, so that both cells and medium may serve as starting material for purification procedures. The structural studies presented here were done on preparations of a Ca antigen isolated from deoxycholate extracts of Hep 2 cells by affinity chromatography on columns of Ca1 antibody coupled to sepharose 4B beads. The antigen eluted from the affinity columns was further purified by high pressure liquid chromatography. The elution pattern of the purified antigen on sepharose 4B columns confirmed its high molecular weight and showed the microheterogeneity characteristic of glycoproteins with a high carbohydrate content. The isoelectric point of the purified antigen determined by elecro-focussing in ampholines was found to be 6.8-7.0. The behaviour of the antigen on DEAE columns

- 6 -

was consistent with an isoelectric pont in this region.

Amino acid analysis of the polypeptide moiety of purified preparations of the Ca antigen confirmed that a large part of the material was carbohydrate. The principal amino acids found were serine, threonine, glycine, alanine and glutamic acid, which together, consistuted more than 50% of the total amino acids relesed. Serine and threonine together constituted about 20%. At least 95% of the carbohydrate was found to be O-glycosidically linked to the polypeptide: trace amounts of N-glycosidically linked carbohydrate could be due to impurities in the preparation. The major sugars present were sialic acid, N-acreylgalactosamine (Gal NAc) and galactose (Gal). The sialic acid was completely susceptible to $\underline{V.}$ $\underline{cholerae}$ neuraminidse and was probably all N-acetylneuraminic acid (Neu NAc). No evidence was obtained for the presence of N-glycolyl neuraminic acid. Treatment of the asialo preparation of the Ca antigen with $\underline{D. \ pneumoniae}$ endo- $\alpha$ - -N-acetylgalactosaminidase indicated that most of the carbohydrate in the asialo-antigen was composed of the disaccharide Gal $\longrightarrow$ Gal NAc $\overset{\alpha}{\longrightarrow}$. Analysis of the

oligosaccaride released from the Ca antigen by alkaline borohydride indicated that there were roughly equal proportions of tetrasaccharide, trisaccharide and disaccharide. The oligosaccharides of the Ca antigen thus consist mainly of (Neu NAc)n⟶Gal⟶Gal NAc⟶, where n = 0, 1 or 2. Examination of the glycopeptides released by treatment of the antigen with pronase suggested that the oligasaccharides occurred as clusters along the polypeptide chain.

The present invention also includes a method of producing an antigen in accordance with the invention by forming a culture of malignant cells, harvesting the cells, extracting with an extractant, separating the extract from the cell residue, fractionating the extractant thus obtained by chromatography, causing or allowing the separated antigen fraction to bind to a suitable binding agent, separating the binding agent and the antigen and eluting the antigen from the binding agent and thereafter purifying the antigen thus obtained.

The extractant may be sodium deoxycholate and the binding agent may be selected from the lectin wheat germ agglutinin; the elution may be performed with

N-acetyl glucosamine in phosphate buffered saline. In an alternative embodiment of the present invention, the extractant may be contacted with an antibody binding agent and thereafter the antigen may be eluted with an elutant to remove the antigen therefrom. The elutant in this case may be an alkali metal isothiocyanate. The binding agent may be coupled to a particulate carrier which is supported in a column for contact with the extractant.

The invention further includes a monoclonal antibody directed at human tumour cells which recognises and binds with the family of Ca antigen, in accordance with the invention. The antibody may be an IgM antibody as determined by Ouchterlony immunodiffusion analysis against a specific immunoglobin u chain antibody. The monoclonal antibody characterised as an IgM antibody may be subjected to splitting techniques to produce an IgG antibody. The antibody in accordance with the present invention liberates on reduction by dithiothreitol a $\mu$ chain having a molecular mass of 72,000 to 74,000 when determined by sodium dodecyl sulphate acrylamide gel electrophoresis.

The present invention further includes a method of testing for the presence of malignant cancer cells in tissue which method comprises

1. preparing a tissue specimen

2. washing the specimen

3. applying a first antibody in accordance with the present invention and allowing it to react with said specimen.

4. removing unbonded antibody

5. reacting the specimen so treated with a secondary agent or antibody which binds with the first antibody.

6. thereafter causing or allowing selected marking or staining of the sites of bonding of the first antibody to the specimen to occur.

The secondary agent may be an antibody directed to the first antibody and the second antibody may be conjugated with the enzyme peroxidase and marking or staining may be effected using chromogenic substrate of the enzyme to produce colour in the sites in which the peroxidase enzyme is localised. The chromogenic substrate may be diaminobenzidine. The tests may include the introduction of control antibodies to the sample for detection of non-specific binding. It is preferred that the first antibody is present in the form of supernatants from cultures in a concentration of 14 μ gram per mil to 16 μ gram per mil.

The invention further includes an immunogen for use in the production of monoclonal antibodies in accordance with the invention which comprises partially purified membrane proteins or groups of proteins obtained from a malignant human cell line which bond to the lectin wheat germ agglutinin. The immunogens in accordance with the invention may be obtained by preparing an extract from a cultured cell line derived from human cancer cells, contacting the extract with the lectin wheat germ agglutinin for a period sufficient to obtain binding of the immunogen thereto, eluting the non-bonded material and subsequently stripping the product immunogens from the lectin wheat germ agglutinin. The non-bonded material may be eluted using Tris-HCL having a pH of the order of 8 and bonded glycoproteins may be stripped subsequently from the lectin wheat germ agglutinin by using N-acetyl D-glucosamine-Tris-HCL at a pH of 8. After stripping from the lectin wheat germ agglutinin, the immunogen may be precipitated with absolute alcohol at a reduced temperature, the precipiation so formed being further washed with phosphate buffered saline.

The monoclonal antibodies in accordance with the present invention may be prepared using such an immunogen by forming a culture of spleen cells from mice which have been immunised with said immunogen, subsequently fusing the cells with mouse mydona cells and promoting growth of the culture, selecting groups of cells from said culture and assaying the same for the presence of antibody binding to a malignant cell line, selecting those groups of cells which show a substantial positive finding and further cultivating the selected groups of cells to produce antibodies and thereafter harvesting and separating the cells and purifying the antibody thus produced. A specific cell line which has been used successfully in accordance with the present invention, is that which has been deposited at the CNCM under No. I-195. The present invention further includes a pharmaceutical composition comprising a pharmaceutically effective derivative of an antibody in accordance with the invention together with a pharmaceutically acceptable diluent and/or carrier therefor.

The antibodies of the present invention may be coupled to radioactive or radioopaque compounds which can themselves be used to locate malignant tumours in a

- 12 -

clinical context. The antibodies of the invention may also be coupled to toxins such as dipheria toxin or ricin, or to cytotoxic subunits of such toxins, or to other cytotoxic or cytolytic agents to produce conjugates that attach specifically to malignant cells and kill them.

The invention further includes a vaccine including an efective proportion of the antigens in accordance with the present invention together with a pharmaceutically acceptable carrier therefor.

Hitherto monoclonal antibodies directed against human tumour cells have usually been produced by immunizing mice with preparations of whole cells. This approach has the disadvantage that while the number of possible antibodies that might be formed is unrestricted, the immune response in the animal itself in practice tends to be dominated by cells responding to a small number of strong antigens.

In the present invention the applicants have sought to increase the chance of isolating the antibodies directed against weak antigens by immunizing the mice with partially purified membrane proteins. The present invention, therefore, is characterised by use of membrane proteins which would bind the lectin wheat germ agglutinin and which have also been partially purified.

The formation of the monoclonal antibodies, apart from this follows substantially the route well known and previously discussed by Kohler and Milstein in Nature 1975; 256: 495-497 and European Journal of Immunology 1976; 6;511-519.

Following is a description by way of example only of methods of carrying the invention into effect.

EXAMPLE 1

Preparation of the immunogen

Extracts were prepared from cultured cells (H.Ep.2) derived from a human laryngeal carcinoma. About $10^8$ were harvested when confluent with 0.02% (w/v) EDTA in PBS. The cells were spun down at 1000 g for 5 min and washed once in PBS. The washed pellet of cells was then taken up to an equal volume of 1% Triton-X100 (v/v)-10 mM Tris-HCl, pH 8.0, and extracted at $4^{\circ}$C for 1 h. The suspension was spun down at 3000 g for 10 min, and the supernatant, to which sodium deoxycholate was added to make a final concentration of 1% (w/v), was incubated with 5 ml of a preparation of wheat germ agglutinin-Sepharose (Pharmacia) in a column for 45 min at 20°C. Material that did not bind to the WGA-Sepharose column was eluted with 50 ml of 10 mM Tris-HCl, pH 8.0. Glycoproteins that bound specifically to WGA were eluted with 20 ml of 0.7 M N-acetyl-D-glucosmine-10 mM Tris-HCl, pH 8.0. The glycoproteins were precipitated with absolute ethanol at -20°C and washed twice with PBS. About 1 mg of glycoprotein that bound specifically to wheat germ agglutinin was obtained from $10^8$ H.Ep.2 cells.

EXAMPLE 2

The immunogen produced in Example 1 was prepared as 500 microgram preparations in complete Freund's adjuvant and injected subcutaneously into 3 month old Balb/C mice. Twelve days later another 250 microgram glycoprotein preparation was injected into each mouse without adjuvant, intraperitoneally, and 7 days later a further 200 microgram was injected by the same route.

The spleen cells were taken from the immunized mice 4 days after the last injection of immunogen. They were fused with P3/NS/1-Ag4 mouse myeloma cells essentially as described by Galfre _et al_ Nature, Lond. 1977; 266 : 550-552. A 1.5 ml volume of 50% (w/v) polyethylene glycol 4000 in PBS was used to promote cell fusion, and all centrifugation steps were done at 600 g for 3 min. After fusion, the cells were suspended in 48 ml of RPMI 1640 medium containing 10% (v/v) foetal calf serum (FCS) and hypoxanthine, aminopterin, thymidine and glycine to make final concentrations of 8, 0.2, 1.2 and 30 mg/l respectively. One ml samples of the cell suspension were dispensed into 2 ml wells in Linbro BCL-5041 plastic trays. To each well 1 ml of a suspension of rat thymocytes, at a concentration of $10^6$ cells/ml, was added to act as a feeder cell population.

Fourteen days after cell fusion, the supernatant from each 2 ml well was assayed for the presence of antibody that bound to H.Ep.2 cells. The assay used was the indirect trace-binding radioimmunoassay described by Williams in "Contemporary Topics", Molecular Immunology 1977; 6: 83-116. A supernatant was scored as positive

- 16 -

if in the radioimmunoassay it gave a value at least twice as high as that given by a negative control consisting of the supernatant from a culture of P3/NS/1-Ag4 myeloma cells alone.

The cells from each well scored as positive were transferred to a 2 ml well in a Linbro BCL-5041 plate together with $10^6$ rat thymocytes and were grown on until the floor of the well was covered with a dense layer of cells. The supernatants from these wells were again assayed from antibody against H.Ep.2 cells by the trace-binding radioimmunoassay. The cells from each well scored as positive were then transferred to a 50 ml plastic culture flask and grown in RPMI 1640 medium containing 10% FCS to which hypoxanthine and thymidine were added in the concentrations given above, but no aminopterin. The antibody thus produced was designated Cal antibody and was subsquently purified.

The hybrid cells producing the Cal antibody were grown within the peritoneal cavity of genetically compatible mice. The antibody accumulated to a very high concentration in the ascitic fluid. (DBA/2 x Balb/c)F mice were injected intraperitoneally with 0.5 ml of pristane and 7-10 days later about $5 \times 10^6$ hybrid cells were inoculted into the ascitic fluid. The contents of the peritoneal cavity were harvested 14-21 days later and the cavity washed out with 5 ml of PBS. The ascitic fluid and the washings were pooled and the cells deposited by low speed centrifugation. The supernatant was then spun at 1300 g for 5 min to separate the pristane droplets which collected in a lipid layer on the surface of the samples. This was aspirated off and the clear solution stored at $-20^{\circ}C$.

The Cal antibody was purified by adding sodium sulphate at room temperature to the clarified ascitic fluid or to concentrated culture supernatant to make a 20% (w/v) solution. The precipitate was allowed to form overnight and was collected by centrifugation at 7,700 g for 30 min at $20^{\circ}C$. The resulting pellet was dissolved in a buffer of 50 mM Tris/HCl at pH 7.4 containing 150 mM KCl and 5 mM $MgCl_2$ and solution was dialyzed against this buffer at $4^{\circ}C$. The retained protein was adjusted to make a concentration of 20-25 mg/ml and the sample was then layered onto a 15-55% (w/v) sucrose density gradient

- 18 -

made up in the same buffer. Centrifugation at 100,000 g for 21-24 h at 20°C resolves two major protein peaks in the gradient, one of high molecular mass containing IgM and $\alpha$-2-macroglobulin and the other of substantially lower molecular mass. The fractions containing the high molecular mass peak were collected and dialyzed at 4°C against 0.01 M sodium phosphate buffer at pH 6.8. The precipitate that forms contains the IgM which is collected by centrifugation at 10,000 g for 30 min at 4°C. The pellet is then washed once in 0.01 M sodium phosphate buffer, dissolved in PBS and stored at -20°C. Densitometer tracings of this material show that it contains less than 5% impurity.

The Cal antibody was shown to be an IgM by Ouchterlony immunodiffusion analysis against a specific rabbit anti-mouse immunoglobulin $\mu$ chain antibody. This was confirmed by sodium dodecyl sulphate acrylamide gel electrophoresis of the Cal antibody after reduction with dithiothreitol. The free $\mu$ chain migrated in the gel in the 73,000 molecular mass position.

EXAMPLE 3

The Cal antibody described above was then subjected to extensive testing to determine its effectiveness or otherwise for use in the characterisation of diagnosis of cancer.

The Cal antibody was tested against the following panel of cells; H.Ep.2, RT112/84, a cell line derived from a human bladder carcinoma, MRC5, a strain of diploid human fibroblasts derived from the lung of a 4 month old foetus, PG19, a cell line dervied from a spontaneous melanoma arising in a C57 black mouse, and secondary cultures of fibroblasts derived from Balb/c mouse embryos.  Table 1 shows the binding radioimmunoassays for three antibodies in this panel of cells.  W6/1 is a monoclonal IgM antibody that binds specifically to the human blood group A antigen and serves as a negative control.  E37 is a monoclonal antibody that binds to all human and mouse cells in culture and serves as a positive control.  Cal binds to the two malignant human cell lines, but not to the diploid human fibroblasts or to the two mouse cell cultures.

- 20 -

TABLE 1. Binding of antibodies to a panel of malignant and non-malignant cells

| Cell line[a] | Species | Progressive growth in vivo[b] | Amount of antibody bound (c.p.m. x $10^{-3}$)[c] | | |
|---|---|---|---|---|---|
| | | | W6/1 | E37 | Cal |
| H.Ep.2 | Human | + | 3.2 | 14.0 | 15.1 |
| RT112/84 | Human | + | 4.2 | 8.4 | 18.3 |
| MRC5 | Human | - | 4.6 | 16.1 | 2.9 |
| PG19 | Mouse | + | 2.8 | 20.3 | 4.3 |
| Balb/c fibroblasts | Mouse | - | 2.9 | 18.6 | 2.4 |

a. For each assay, about $2 \times 10^5$ viable target cells were incubated with the two antibodies as described by Williams Contemp Topics molec Immund 1977; 6:83-116.

b. Human cells assayed in nude mice; mouse cells in sublethally irradiated, syngeneic new-born mice.

c. The values given are the means of duplicates in the

binding radioimmunoassay. About 300,000 cpm of $^{125}$I-labelled second antibody were added to each microtitre well.

EXAMPLE 4

A further series of tests consisted of a panel of matched pairs of hybrid cells derived from crosses between cells of human cervical carcinoma (D98AH-2) and diploid human fibroblasts (S1814 or GM1604). One member of each pair was a hybrid in which malignancy was suppressed, the other a malignant segregant, malignancy being assessed by progressive growth in the nude mouse. Each pair of hybrids was independently derived, and the panel was assembled from two different laboratories.

Table 2 shows the binding of the Cal antibody to the panel of such matched pairs of hybrid cells. W6/1 again serving as a negative control, and the positive control being provided by W6/32, a monoclonal antibody with anti-HLA specificity.

- 22 -

TABLE 2. Binding of antibodies to panel of matched pairs of hybrid cells

| Cell line | Growth in nude mouse | Amount of antibody bound $(c.p.m. \times 10^{-3})$[c] | | |
|---|---|---|---|---|
| | | W6/1 | W6/32 | Ca1 |
| 1Acn2[a] | - | 3.4 | 79.0 | 2.5 |
| 1Acn1TG[a] | + | 2.0 | 78.1 | 23.1 |
| 2B1coll[a] | - | 2.6 | 60.0 | 2.2 |
| 5Amc3[a] | + | 2.5 | 74.2 | 16.4 |
| 4-4-4[b] | - | 2.4 | 78.2 | 2.6 |
| 541E[b] | - | 3.3 | 81.3 | 3.3 |
| 541M[b] | + | 2.2 | 56.6 | 14.1 |
| CG04[b] | + | 3.1 | 90.1 | 51.0 |
| CGL3[b] | + | 1.9 | 68.2 | 39.4 |

a. D98AH-2 x S1814 diploid human fibroblast crosses supplied by Dr. Harold Klinger, Dept. of Genetics, Albert Einstein College of Medicine, Bronx, N.Y.,

U.S.A.

b.  D98AH-2 x GM1604 diploid human fibroblast crosses supplied by Dr. Eric Stanbridge, Dept. of Microbiology, College of Medicine, University of California, Irvine, Calif., U.S.A.

c.  Conditions of the assay are as shown in Table 1.

From the foregoing it is clear that the Cal antibody binds to all the malignant segregants but does not bind to any of the hybrids in which malignancy is suppressed.

EXAMPLE 5

The results from Tables 1 and 2 above indicate that the antigen recognised by Cal antibody is  present on malignant cells derived from laryngeal carcinoma, a bladder carcinoma and a cervical carcinoma.  In order to determine the extent of the presence of this antigen on other human malignant cell lines the binding of the Cal antibody to a range of such cell lines and to some additional non-malignant diploid human cells is shown in Table 3.  W6/1 and W6/32 again serve as negative and positive controls respectively.

TABLE 3. Binding of antibodies to a range of malignant human cells and to non-malignant human cell strains.

| Cell line | Origin | Growth in nude mouse | Amount of antibody bound (c.p.m. x $10^{-3}$) [a] | | |
|---|---|---|---|---|---|
| | | | W6/1 | W6/32 | Cal |
| H.Ep.2. | Laryngeal carcinoma | + | 3.1 | 35 | 30 |
| RT112/84 | Bladder carcinoma | + | 4.3 | 58 | 24 |
| EJ138 | Bladder carcinoma | + | 4.2 | 16.7 | 10.8 |
| T24/83 | Bladder carcinoma | + | 4.2 | 19 | 14.5 |
| H29/219 | Rectal carcinoma | + | 3.7[b] | 35 | 15 |
| RPM12650 | Nasal septum carcinoma | + | 3.2[b] | 90 | 20 |
| HT55F | Colon | | | | |

|  | | | | | |
|---|---|---|---|---|---|
|  | carcinoma | + | 0.4 | 2.9 | 7.1[c] |
| Morwood | Bronchial carcinoma | + | 2.5 | 11 | 25 |
| Ben | Bronchial carcinoma | + | 3.2 | 30 | 19 |
| HeLa (Flow Labs) | Cervical carcinoma | + | 4.4 | 40 | 36 |
| Daudi | Burkitt lymphoma | - | 4.1 | 4.1[d] | 3.4 |
| MRC5 | Foetal lung fibroblast | - | 4.6 | 21 | 2.9 |
| W138 | Foetal lung fibroblast | - | 3.2 | 29 | 3.4 |
| S1814.PB5 | Foetal fibroblast | - | 2.5 | 51 | 3.2 |
| Lymphocytes | Normal peripheral blood | - | 3.5 | 24 | 2.6 |

a. Conditions of assay as shown in Table 1.

b. Second antibody only: these cells bind W6/1.

c. $10^5$ target cells. - 26 -

d. Does not express surface HLA antigens, and does not grow in the nude mouse.

It is clear that Cal antibody detects an antigen that is present on the human malignant cell lines that grow progressively in the nude mouse, but not on the non-malignant cell strains or normal peripheral blood lymphocytes.

EXAMPLE 6

In view of the results from Example 5 experiments were carried out to test the binding of Cal antibody to normal human tissues. Since rather few human tissues are available as cell strains that grow in vitro , the binding of Cal antibody to a range of normal human tissues was measured by the ability of a homogenate of each tissue to inhibit a trace-binding radioimmunoassay of the antibody with H.Ep.2 cells as targets. Details of the inhibition assay are given in Gingrich et al in Immunological delineation in normal and malignant cells of a membrane protein involved in glucose transport.

1. Preparation and properties of the antibody. J Cell Sci 1981; 52: 99-120. Adult human tissues were obtained from a previously healthy 40 year old accident victim who had been maintained on a life support system as an organ donor. Human foetal tissues came from a 16 week pregnancy electively aborted by prostagladin infusion. All procedures conformed to the guidelines for patient protection established at the John Radcliffe Hospital, Oxford. Table 4 shows the amount of Cal antibody bound by a range of normal tissues expressed as a percentage of the amount bound by H.Ep.2 cells. The results are calculated on the basis of protein content. It will be seen that the normal adult and foetal tissues bind very small amounts of the antibody compared with H.Ep.2 cells.

TABLE 4.  Binding of Cal antibody to normal human adult and foetal tissues

| Adult tissue | Absorptive capacity (per cent) | Foetal tissue | Absorptive capacity (per cent) |
|---|---|---|---|
| H.Ep.2 | 100 | | |
| Lung | 3-10 | Lung | 8 |
| Small intestine | 2 | Small intestine | 5 |
| Heart | 2 | Heart | 2 |
| Spleen · | 2 | Kidney | 2 |
| Brain | 0 | Brain | 2 |
| Liver | 2 | Liver | 2 |
| Skeletal muscle | 2 | Skeletal muscle | 3 |

The antibody described above can, therefore, be used in a standard indirect immunoperoxidase test, as described by Nakane P.K. and Kawaoi A. in the Journal of Histochemistry and Cytochemistry 1974; 22 : 1084-1091 which permits the Ca antigen to be localised in fixed preparations of cells and tissues.  The reaction may be carried out in frozen sections as used routinely in biopsy material, or because of the resistance of the Ca antigen to organic solvents, on standard paraffin sections. From the foregoing, it is apparent that Ca antigen is present on the vast majority of malignant human tumours but not on the cells of benign tumours or on normal tissue cells. Non-specific interactions of low affinity between the Cal antibody and some tissue constituents such as mucin or keratin, do not present a serious technical problem. The antibody, therefore, provides a valuble diagnostic application for the presence of malignant cells in samples.

EXAMPLE 7

The diagnosis of the presence of cancer cells in a sample using the antibodies in accordance with the invention is carried out by the staining of fixed, paraffin-embedded freshly cut tissue sections. Acid fixatives should be avoided; formalin fixatives should for preference be stored over marble chips to neutralise acid formed on keeping. Post-fixation in mercury salts does not inhibit staining. Cryostat sections should be fixed in acetone at minus 20°C. No information is yet available concerning the preservation of antigen in post mortem tissues.

The reagents employed are:-

1. <u>Hydrogen peroxidase solution</u> 30 per cent (sigma). Store at $4^{\circ}$C. Discard any remaining 3 months after opening bottle for the first time. Wrap cap and neck of bottle in parafilm after re-closing.

2. <u>Trypsin</u> (sigma, Type IX)> Store at $4^{\circ}$C.

3. <u>Phosphate buffered saline</u> (PBS)

| | |
|---|---|
| Potassium dihydrogen phosphate $KH_2PO_4$ | 0.41 g |
| Disodium hydrogen phosphate $Na_2HPO_4$ | 1.00 g |
| Sodium chloride NaCl | 8.50 g |
| Distilled water to | 1.0 litre |

Check and adjust pH to 7.2 - 7.4

Sterilise by autoclaving or membrane filtration.

4. <u>Buffered Trypsin</u>

| | |
|---|---|
| Trypsin | 50 mg |
| PBS | 10 ml |

Make up only as much as is required for immediate use. Discard within three hours.

5.  <u>Methanol/Peroxide</u>

    Absolute methanol                                    18 ml

    Hydrogen peroxidase solution                          2 ml

    Prepare freshly for use.  Discard after 4 hours.


6.  <u>Solution A</u>

    Foetal calf serum                                   100 ml

    Bovine albumin powder (sigma)                        100 g

    Sodium azide                                           1 g

    PBS                                      to 1 litre

    Store at 4°C for up to 3 months.  Discard if
    visibly contaminated.  (For convenience, Bovine
    Albumin 30% solution, 333 ml, may be substituted for
    the powder).


7.  <u>Solution B</u>

    Solution A                                           95 ml

    Human serum (group AB)                                5 ml

    Store at 4°C for up to three months.  Discard if
    visibly contaminated.


8.  <u>Ca 1 Antibody Solution</u>      (Commercially available
    from Wellcome Diagnostics under the reference RP.82).
    Dissolve the contents of one vial with 0.5 ml of distilled water. Store for up to one week at 4°C or for up to one month frozen at -20°C. Do not re-freeze after thawing. Any further dilution for use should be made in Solution A.

9. <u>Conjugate Solution</u> (working strength)

Dilute the stock peroxidase-labelled anti-mouse Ig solution to the required strength with Solution B. Prepare in small quantities. Solution should be discarded at the end of the working day.

10. <u>Substrate Solution</u>

Diaminobenzidine                                              5 mg

PBS                                                              10 ml

Hydrogen Peroxidase Solution 50 Micro (2 drops) Store at 4°C

The diaminobenzidine solution may be prepared when convenient on the day of use. The peroxide should be added immediately before use and unused material discarded within a few minutes.

The Samples are prepared as follows:-

1. Dewax sections, dry overnight at 37°C. (to ensure adequate adhesion) and take through to water in the usual way.

2.   Place slides, tissue upward, in a moist chamber in an incubator at 37°C.

3.   Apply sufficient Buffered Trypsin to cover the tissue section generously.  Close the chamber and incubate at 37°C for 30 minutes.

4.   Wash the slides in three changes of PBS for 10 minutes each change at room temperature.

5.   Immerse slides in Methanol/Peroxidase for 30 minutes at room temperature.

6.   Wash slides in three changes of PBS for one or two minutes each change at room temperature.

7.   Immerse slides in Solution A for 30 minutes at room temperature.

8.   Drain slides and carefully dry with lint-free paper around the area of the tissue sections.

9.   Place slides horizontally, tissue upward, in a moist chamber.

10.  Apply sufficient Cal antibody solution to cover the tissue section generously.  Close the chamber  and incubate at room temperature for 60 minutes.

11.  Wash the slides with three changes of Solution A, each for ten minutes.

12.  Drain and dry slides and replace in moist chamber as in steps 8 and 9.

13. Apply sufficient working-strength Conjugate Solution to cover the tissue section generously. Close the chamber and incubate at room temperature for 30 minutes.

14. Wash the slides with three changes of PBS, each for two or three minutes. Drain the slides.

15. Flood the tissue sections with Substrate Solution, close the chamber and incubate at room temperature for 5 minutes.

16. Wash·slides with slowly running tap water for 5 minutes.

17. Counterstain tissue sections lightly with Harris' haematoxylin, dehydrate, clear and mount for microscopic examination.

EXAMPLE 8

A number of samples of human tissues including carcinomas of skin, tongue, larynx, lung, oesophagus, stomach, intestines, liver, kidney, bladder, breast, uterus, ovary and thyroid, were all prepared by fixing in 10% (v/v) formaldehyde in 0.15 M NaCl (pH 7.0) for 12-48 hours depending on the size of the tissue block.

The block is then processed in the following sequence of reagents:-

1. 70% (v/v) ethanol + 10% (v/v) formaldehyde in water for a period of 1 hour

2.   100% ethanol for a period of 2 hours

3.   100% ethanol for a period of 1 hour

4.   100% ethanol for a period of 1 hour

5.   100% ethanol for a period of 1 hour

6.   100% ethanol for a period of 1 hour

7.   100% ethanol for a period of 1 hour

8.   chloroform for a period of 1.5 hours

9.   chloroform for a period of 1.5 hours

10. chloroform for a period of 1.5 hours

11. paraffin wax for a period of 2.5 hours

12. change of paraffin wax for a period of 2 hours


The paraffin sections were cut at 80μm and incubated on glass slides for 16 hours at 37°C.


After preparation the paraffin sections were dewaxed by passage through xylene and an alcohol series to water in the standard way.  The dewaxed sections were then immersed in phosphate-buffered saline (PBS) at pH 7.3 for 5 min.  The sections were then transferred to PBS containing 10% (v/v) foetal calf serum and 10% (v/v) bovine serum albumin (FB medium) for 30 minutes.

After this treatment the sections were then reacted for 60 minutes with the culture supernatant containing the Cal antibody and diluted 1 in 2 with FB medium. The culture supernatants containing the control G6 and CR2 antibodies were reacted with the sections in the same way. The sections were washed in Fb medium and then immersed for 60 minutes in PBS containing the second antibody (a rabbit anti-mouse Ig conjugated with peroxidase) at a dilution of 1 in 30 together with 5% (v/v) human serum (group O) and 5% (v/v) foetal calf serum.

The sections were then washed for two periods of 15 minutes and two changes of PBS and then reacted for 5 minutes with a solution of diaminobenzidine to a concentration of 50ug/l containing 0.01% (v/v) $H_2O_2$. The sections were then washed in tap water and stained with haematoxylin. The sections were mounted in DPX.

Non-specific binding of the Cal antibody in the method described above was observed in keratin and keratin-containing cells, in the mucus of some parts of the gastro-intestinal tract and ovary and in the collecting tubes of the kidney and in sweat glands.

These non-specific reactions did not pose problems in diagnosis. The binding of the IgM antibodies to these sites was of rather low affinity and if necessary could be largely eliminated by reacting the antibody with the tissue section at a higher ionic strength (up to 1.5 M NaCl) than the standard conditions given in the above Example. At this higher ionic strength, specific binding of Cal antibody to the tumour cells in virtually unaffected. Specific, high-affinity binding of Cal antibody was not observed in any normal cells or tissues.

The experiment described above was further carried out with a range of benign tumours of different kinds (adenomas, fibromas, fibroadenomas, leiomyomas) at different sites. None showed specific binding of Cal antibody.

A wide range of malignant tumours were prepared as described in the above Example and the collection represents something approaching 85 to 90% of all malignant tumours known in man. The sections were cut so that normal and cancerous tissue were present in each section, thus serving as an internal control. For several of the carcinomas, the corresponding benign tumour was available for comparison. The vast majority of these carcinomas expressed the Ca antigen and as a result a colour reaction developed for each tumour. The amount of Ca antigen appeared to vary from tumour to tumour as measured by the development of the colour reaction and in some tumours there was a marked patchiness in the intensity and distribution of the colour reaction due to focal staining.

Of the carcinomas investigated in the above Example, however, all of those of the Example quoted above showed a positive reaction showing clearly that all these carcinomas express the Ca antigen and thus providing a positive diagnostic test for malignant tumours.

A further use of the Cal antibody produced as described above is in the identification of malignant cells in smears and exudates. This is sometimes very difficult and is facilitated by the use of the immunoperoxidase test to detect the presence of the Ca antigen. This is particularly useful in smears of the uterine cervix since, as is shown above, cervical cancer cells do produce the Ca antigen.

The Cal antibody can also be used to construct a radioimmunoassay for blood or other body fluids from patients with malignant tumours if the malignant tumour cells or antigen are shed into the body fluids. A small number of normal tissues showed non-specific binding of the Cal antibody, that is, binding that was also seen with the control IgMs. These tissues are the epidermis and sebaceous glands of the skin, some intestinal mucins, the collecting tubules of the kidney and the cervical epithelium of the uterus. The binding of the Cal antibody at these sites could be abolished if, before being processed through the immunohistochemical procedure, the dewaxed paraffin sections were treated with trypsin and endogenous peroxidase activity was blocked with methanol/$H_2O_2$.

Treatment with 0.5% (w/v) trypsin(sigma) in PBS for 30 min and with methanol containing 10% $H_2O_2$ for 30 min eliminated this non-specific binding, but enhanced the rection of the Cal antibody with mammary cancers.

EXAMPLE 9

Characterisation of the antigen

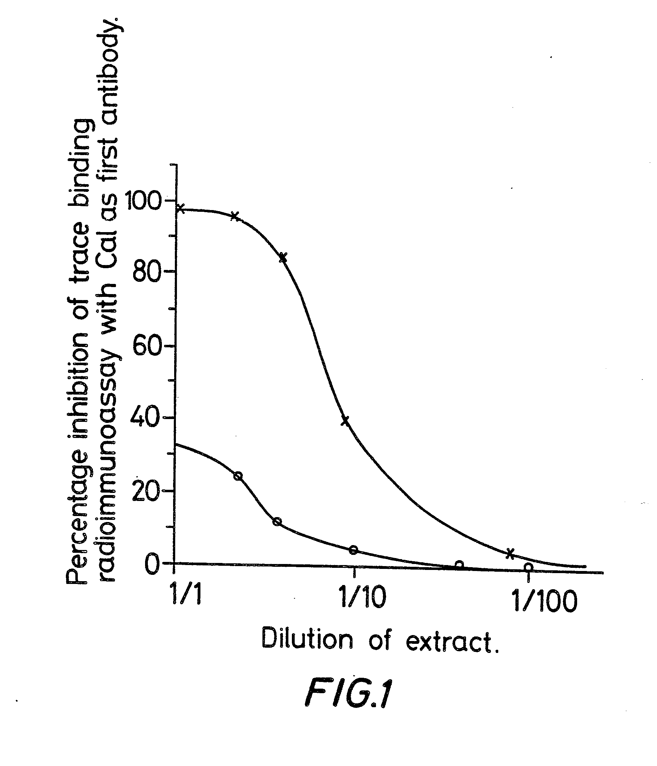

Figure 1 of the accompanying drawings is a graph showing the percentage inhibition of trace binding radioimmunoassay with Cal as first antibody against dilution of extract.

Immunoprecipitation experiments with 0.2% (w/v) DOC extracts of a range of malignant and non-malignant cells, including matched pairs of hybrid cells, revealed that the Cal antibody specifically precipitated from the malignant cells and the malignant partners of the matched pairs of hybrid cells two components which, both before and after reduction by dithiothreitol, separated in SDS acrylamide gels as bands with estimated molecular masses of 390,000 and 350,000. No such components were precipitated by Cal from non-malignant cells or the non-malignant partners of the matched pairs of hybrid cells. These components appeared to be glycoproteins with a very high carbohydrate content. They could be readily labelled by incubating malignant cells in [$^{14}$C] - glucosamine, and, with greater difficulty, by incubating them in a [$^{14}$C] - protein hydrolysate, but the bands stained poorly with Coomassie Blue. Affinity labelling with [$^{125}$I] - wheat germ agglutinin showed that both the bands resolved in the SDS gels bound this lectin. Fractionation of a DOC extract of H.Ep2 cells by high performance liquid chromatography confirmed that a fraction containing the 390,000 and 350,000 molecular mass components contained the antigen, as measured by its ability to inhibit the binding of the Cal antibody to target cells in a radioimmunoassay The two components could be partially resolved by centrifugation in a 10 to 40% sucrose density gradient. Analysis of appropriate fractions from the gradient by radioimmunoassay showed that the Cal antibody bound specifically to both the 390,000 and the 350,000 molecular mass components. After initial fractionation by high performance liquid chromatography, these two components could be further purified by affinity chromatography on a column of wheat germ agglutinin - Sepharose. An affinity column made by coupling the purified Cal antibody to cyanogen bromide-activated Sepharose 4B beads (Pharmacia) could be used to isolate the antigenic components directly from DOC extracts of malignant cells.

The functional relationship between the two antigenic components remains to be explored. The relative amount of each varies from one cell type to another, but the 390,000 molecular mass component usually predominates. The 350,000 molecular mass component does not, however, appear to be a degradation product of the larger component. The presence of protease inhibitors at all stages of the isolation and fractionation procedures had no significant influence on the ratio of the two components in any one preparation; and if proteolysis was deliberately permitted to occur, the smaller component did not increase in amount at the expense of the larger.

Examination of the stability of the antigen under a variety of conditions revealed that it was extremely robust. The antigen is completely resistant to boiling at $100^\circ C$ for up to 30 min and is not destroyed, or extracted from the cells, by organic solvents such as ethyl alcohol, methanol, xylol, or chloroform-methanol mixtures. Since rather few protein antigens survive such drastic procedures, whereas polysaccharide antigens commonly do so, the possibility that the specific determinant might reside in the polysaccharide moiety of the antigen was explored.

It was found that treatment with neuraminidase prepared from either V. cholerae (BDH or Calbiochem-Behring, the latter said to be protease-free) or from influenza virus (Calbiochem-Behring) greatly reduced the ability of glutaraldehyde-fixed tumour cells to bind Cal antibody.

This was also true for DOC or Triton X-100 extracts of tumour cells. The extent of reduction varied somewhat from one preparation to another but was usually in the region of 60% after 2 hours incubation with the enzyme at 37°C. The effect of neuraminidase was readily demonstrable in cell extracts clarified by boiling. Boiling the extract for 5 minutes rsulted in the denaturation of most of the proteins in it and these could then be removed by centrifugation. The antigen that bound the Cal antibody remained intact in the supernatant. Figure 1 shows the effect of V. cholerae neuraminidase on the binding of the Cal antibody to an extract of HEp.2 cells clarified by boiling. Treatment of tumour cell extracts with neuraminidase reduced the binding of $[^{125}I]$ - wheat germ agglutinin to the 390,000 and 350,000 molecular mass components resolved in the SDS acrylamide gels. These two components could not be immunoprecipitated by the Cal antibody from tumour cell extracts that had been treated with neuraminidse. The sensitivity of the antigen to neuraminidase indicated that terminal sialic acid residues of the polysaccharide moieties of the glycoproteins are involved in the binding of Cal to the antigen.

Treatment of the tumour cell extract with a preparation of mixed glycosidases containing both exo- and endo-glycosidases but no neuraminidase activity (Miles Biochemcials) also reduced the ability of the extract to bind the Cal antibody.

The electrophoretic mobility of the antigen was unaffected by chondroitin ABC lyase (4.2.2.4), chondroitin AC lyase (4.2.2.5), chondro-4-sulphatase (3.1.6.9) and hyaluronidase (3.2.1.35) (all from Miles). The binding of the Cal antibody to the antigen was, however, greatly reduced by keratan sulphate endo-$\beta$-galactosidase (Miles) which released the carbohydrate moiety of the antigen as oligo-saccharide chains. But the antigen is not a keratan sulphate as the endogalactosidase does not release the N-acetyllactosamine disaccharide characteristic of keratan sulphate; nor does the antigen incorporate radioactively labelled sulphate.

Terminal sialic acid residues form part of the antigenic determinants of the M and N blood groups which, like the antigen detected by Cal, are resistant to high temperatures and the common organic solvents. Cal was therefore tested against a panel of human red cells from 36 different blood groups including M and N. The antibody, at concentrations of 6.1, 0.61 and 0>061 ug/ml, was tested directly and by the albumin and papain enhancing techniques against suspensions of the erythrocytes in physiological saline at $37^{\circ}$C. A, $A_2$ and B group erythrocytes were also tested at $12^{\circ}$C. No positive reactions were observed. Despite the superficial similarities described above, the antigen detected by Cal thus differs from the M and N blood group antigens as they occur on erythrocytes. It also differs from the T and Tn antigens that have been described in certain carcinomas and are related to the M and N antigens. T and Tn are not sensitive to neuraminidase: indeed their presence may be revealed by the removal of terminal sialic acid residues.[23]

In order to explore whether protein was involved in the interaction of the Cal antibody with its antigen, tumour cell extracts were subjected to proteolytic procedures before assay. Overnight treatment of a tumour cell extract at $37^{\circ}C$ with pronase (Protease Type XIV from Streptomyces griseus , Sigma) virtually eliminated its ability to bind the Cal antibody. It appears, therefore, that protein as well as polysaccharide is involved in the binding of Cal to its antigen.

The antigen is not extracted from cells by PBS or by PBS containing 0.02% (w/v) EDTA> Urea at a concentration of 0.1 M is about 10% as effective and 2 M KCl less than half as effective as 0.1% (w/v) DOC in extracting the antigen from cells. The antigen thus appears to be an integral membrane protein.

In summary, the antigen detected by Cal has the following properties:

1. It is extracted by detergents from the cell membrane as two components that migrate in SDS acrylamide gels with estimated molecular masses of approximately 390,000 and 350,000.

2. These components have the properties of glycoproteins with high carbohydrate content.

3. The antigenic determinant against which Cal is directed is present on both of these components.

4. The antigenic determinant resists boiling and extraction by a variety of organic solvents.

5. The terminal sialic acid residues of the polysaccharide moieties of the glycoproteins appear to form part of the antigenic determinant, for the binding of Cal to the antigen is greatly reduced by treatment of the antigen with neurminidase.

6. The antigenic determinant appears to be destroyed by extensive proteolysis.

7. The antigens of the present invention have the typical features of mucus glycoproteins.


EXAMPLE 10

The starting material for the preparation of the Ca antigen is a monolayer culture of human malignant cells, for example, H.Ep.2, grown to confluence in glass roller bottles under standard conditions. A convenient number of cells for a small scale

preparation is $10^8$.

The cells are harvested in PBS conaining 0.02% (w/v) EDTA and spun down at 2000g for 5 minutes. The cell pellet is washed twice in PBS. The packed cells have a volume of about 1 ml. An equal volume of 0.2% (w/v) DOC-10 mM Tris-HCl, pH 8.0, is added and the pellet of cells dispersed.

The cells are extracted at room temperature for 15 minutes with frequent mixing. The suspension is then centrifuges at 3000 g for 10 minutes and the pellet discarded. The supernatant, which contains the bulk of the cell membrane glycoproteins, is further clarified by centrifugation at 9000g for 2 minutes and is then fractionated by high performance liquid chromatography (HPLC).

A Waters Associates HPLC system was used. This was composed of a Model 6000A solvent delivery system, a UK 6 injector with a 2 ml injection loop, a Model 450 variable wave length detector and a Model 730 data module. Two Waters Associates I-250 gel filtration columns, each 3000 mm x 7.5 mm, were used in the purification procedure. The columns were coupled in series and protected from contamination by a guard column packed with Waters Associates I-125 gel filtration medium. A volume of 250 ul of the clarified DOC extract was applied to the system. The pre-equilibrated columns were then eluted with a complex buffer consisting of 100 mM Tris-HCl, 10% (v/v) glycerol, 0.5 M $Na_2SO_4$, 0.25% (w/v) DOC and 5% (v/v) isopropyl alcohol, at pH 9.0. The buffer was filtered through a 22 micron millipore filter and degassed before use. The solvent uptake tube was fitted with a 2.0 micron filter. The columns were eluted at a rate of 1 ml/min at 1000 psi and the eluate monitored at 230 nm.

Excluded material emerged from the columns after 9.00 min. The Ca antigen emerged at the leading edge of the first peak with a retention time of 9.00-10.50 min.

The HPLC fractions containing antigenic activity, as mesured by their ability to inhibit the binding of Cal antibody to target cells in a radioimmunoassay, were pooled and applied to a 5 ml column of WGA-Sepharose pre-washed with PBS. Binding was allowed to take place for 30 minutes at room temperature. Non-bound material was eluted by passing 5 column volumes (25 ml) of PBS through the column. Material bound specifically to WGA was eluted by passing 10 ml of 0.5 M N-acetylglucosamine in PBS through the column. The elutant was dialyzed against de-ionized water for 24 hours and then lyophilized. A yield of 5 ug of Ca antigen was obtained.

## EXAMPLE 11

The purification of the antigen was carried out using the Ca1 antibody. About 10 milligrams of Ca1 antibody were coupled to 2 grams of cyanogen bromide-activated Sepharose 4B beads (supplied by Pharmacia) by a standard well-known procedure. The malignant cells were extracted with sodium deoxycholate as described in Example 9. The extract was passed through a 5 millilitre column of the Ca1-Sepharose beads and the unbound material is discarded. Material bonding to the column non-specifically was removed by extensive washing with 5 column volumes (25 ml) of 2 M sodium chloride and phosphate-buffered saline solution.

The Ca antigen bound specifically to the column was then eluted with a 3 M potassium isothiocyanate solution (10 ml).

Purified preparations of the Ca antigen described above are useful to generate subsequent generations of antibodies in accordance with the invention.

0087898

- 1 -

CLAIMS

------

1. An antigen derived from malignant cells characterised by

(i) a molecular mass within the range of 340,000 to 400,000,

(ii) the ability to bind to the lectin wheat germ agglutinin,

(iii) resistance to boiling,

(iv) resistance to destruction and to extraction from malignant cells using solvents selected from ethyl alcohol, methanol, xylol and chloroform-methanol mixtures.

2. An antigen as claimed in claim 1 characterised by

(v) being destructable on digestion with pronase,

(vi) a molecular mass component of 350,000 and 390,000,

(vii) a glycoprotein structure with a high carbohydrate content,

(viii) having an isoelectric point of 6.8-7.0.

3.     An antigen as claimed in claim 2 characterised in that amino acid analysis yields, serine, threanine, glycine, alanine, and glutaminic acid which account for more than 50% of the total amino acids released, and in that serine and threanine constitute 18% to 22% of said total and further characterised by a high carbohydrate content in which at least 95% of the carbohydrate is O-glycosidically linked to polypeptide and by the major sugars present are sialic acid, N-acetylgalactosamine and galactose.

4.     A method of producing the antigen claimed in claim 1 characterised forming a culture of malignant cells, harvesting the cells, extracting with an extractant, separating the extract from the cell residue, fractionating the extractant thus obtained by chromatography, causing or allowing the separated antigen fraction to bind to a suitable binding agent, separating the binding agent and the antigen and eluting the antigen from the binding agent and thereafter purifying the antigen thus obtained.

5.     A method of producing an antigen in accordance with claim 4 characterised in that

(i)     the binding agent is the lectin wheat germ agglutinin,

(ii)    the elution is performed with N-acetyl glucosamine in phosphate buffered saline,

(iii)   the extractant is an antibody binding agent,

(iv)    the elutant is an alkali metal isothiocyanate.


6.      A monoclonal antibody directed at human tumour cells characterised in that it recognises and binds with the family of antigens as claimed in claim 1.


7.      A monoclonal antibody as claimed in claim 6 characterised in that on reduction with dithiothreitol a u chain is liberated having a molecular mass of 72,000 to 74,000 when determined by sodium dodecyl sulphate/arylamide gel electrophoresis.


8.      A method of testing for the presence of the malignant cancer cells in tissue characterised in that said said method comprises

(i)     preparing a tissue specimen,

(ii)    washing the specimen,

(iii)   applying a first antibody as claimed in claim 7 and allowing it to react with said specimen, (iv)    removing unbound antibody,

(v)     reacting the specimen so treated with a second reagent or antibody which binds with said first antibody,

(vi)    thereafter causing or allowing selective marking or staining of the sites of binding of the first antibody to occur.


9.      A method as claimed in claim 8 further characterised in that

(vii)   the second reagent is a second antibody directed against said first antibody,

(viii)  said second antibody is conjugated with the enzyme peroxidase,

(ix)    the marking or staining is effected with a chromogenic substrate of said enzyme to produce a colour at the sites where the peroxidase enzyme is localised.

(x)     control antibodies are included for the detection of non-specific binding.


10.     An immunogen for use in the production of monoclonal antibodies in accordance with the present invention which immunogen comprises partially purified membrane proteins or groups of proteins obtained from a malignant human cell line and which bonds to the
lectin wheat germ agglutinin.

11. A method of producing immunogens which
comprises preparing an extract from a cultured cell
line derived from human cancer cells, contacting the
extract with the lectin wheat germ agglutinin for a
period sufficient to obtain binding of the immunogen
thereto, eluting the non-bonded material, and
subsequently stripping the product immunogens from the
lectin wheat germ agglutinin.

12. A method for the preparation of monoclonal
antibodies using the immunogens claimed in claim 10
characterised by forming a culture of spleen cells from
mice which have been immunised with said immunogens,
subsequently fusing said cells with mouse myeloma cells
and promoting growth of the culture, selecting groups of
cells from said culture and assaying for the presence of
antibody binding to a malignant cell line, selecting
those groups of cells which show a substantial positive
binding and further cultivating the selected groups of
cells to produce antibodies and thereafter harvesting
and separating the cells and purifying the antibody thus
produced.

- 6 -

13.    A cell culture medium for use in the preparation
of the monoclonal antibody claimed in claim 6
characterised by that deposited at the CNCM under No.
1-195.

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | NATURE, vol. 286, 28 August 1980, pages 888-891, New York USA; M.B.OMARY et al.: "Human cell-surface glycoprotein with unusual properties" *Page 888, right hand column, lines 5-10; page 889, right hand column, line 14 - page 890, left hand column, line 6* | 1-12 | C 07 G 7/00 <br> C 12 P 1/00 <br> C 12 N 15/00 <br> G 01 N 33/54 <br> C 12 N 5/00 // <br> A 61 K 39/00 <br> A 61 K 39/395 <br> C 12 R 1/91 |
| | --- | | |
| A | US-A-4 174 385 (R.H.REID) <br><br> *Column 2, lines 12-48; column 6, line 61 - column 7, line 32* | 1-5,8-11 | |
| | --- | | |
| A | US-A-4 297 338 (J.G.MAKARI) <br> *Column 2, line 29 - column 3, line 38; column 5, lines 36-53* | 1-3 | |
| | --- | | **TECHNICAL FIELDS SEARCHED (Int. Cl. ³)** |
| A | US-A-4 086 217 (H.J.HANSEN) <br> *Claims; column 10, lines 21-50* | 1-3 | C 07 G <br> C 12 P <br> C 12 N <br> A 61 K |
| | --- | | |
| A | PROC.NATL.ACAD.SCI., vol. 77, no. 1, January 1980, pages 563-566, New York USA; R.S.ACCOLLA et al.: "Monoclonal antibodies specific for carcinoembryonic antigen and produced by two hybrid cell lines" *The whole article* | 1-3,6,7,10,12 | |
| | ---     -/- | | |

The present search report has been drawn up for all claims

| Place of search | Date of completion of the search | Examiner |
|---|---|---|
| THE HAGUE | 24-05-1983 | RYCKEBOSCH A.O.A. |

| Category | Citation of document with indication, where appropriate, of relevant passages | Relevant to claim | CLASSIFICATION OF THE APPLICATION (Int. Cl. ³) |
|---|---|---|---|
| A | US-A-4 172 124   (H.KOPROWSKI) | 1-3,6, 7,10, 12 | |
| | ----- | | |

TECHNICAL FIELDS
SEARCHED (Int. Cl. ³)

The present search report has been drawn up for all claims

| Place of search<br>THE HAGUE | Date of completion of the search<br>24-05-1983 | Examiner<br>RYCKEBOSCH A.O.A. |
|---|---|---|

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1503. 03.82